(19) Europäisches Patentamt
European Patent Office
Office européen des brevets

(11) EP 0 582 209 B1

(12) **EUROPÄISCHE PATENTSCHRIFT**

(45) Veröffentlichungstag und Bekanntmachung des Hinweises auf die Patenterteilung:
**08.04.1998 Patentblatt 1998/15**

(51) Int. Cl.$^6$: **C07C 217/08**, C07C 229/12, C07C 229/22, C07D 207/44, C07D 207/40, C07D 207/26, B01F 17/00

(21) Anmeldenummer: **93112111.5**

(22) Anmeldetag: **29.07.1993**

(54) **Alkoxylierungsprodukte und ihre Verwendung als Dispergatoren**

Alkoxylation products and their use as dispersion agents

Products d'alkoxylation et leur utilisation comme agents de dispersion

(84) Benannte Vertragsstaaten:
**AT BE CH DE ES FR GB LI NL**

(30) Priorität: **03.08.1992 DE 4225619**

(43) Veröffentlichungstag der Anmeldung:
**09.02.1994 Patentblatt 1994/06**

(73) Patentinhaber:
**BASF Aktiengesellschaft**
**67063 Ludwigshafen (DE)**

(72) Erfinder:
- **Ter Maat, Johan Herman Hendrik**
  **D-6800 Mannheim 1 (DE)**
- **Meyer, Marion, Dr.**
  **D-6800 Mannheim 1 (DE)**
- **Oppenlaender, Knut, Dr.**
  **D-6700 Ludwigshafen (DE)**
- **Zirnstein, Walter**
  **D-6905 Schriesheim (DE)**
- **Denzinger, Walter**
  **D-6720 Speyer (DE)**
- **Franz, Lothar, Dr.**
  **D-6704 Mutterstadt (DE)**

(56) Entgegenhaltungen:

| | |
|---|---|
| **EP-A- 0 107 089** | **EP-A- 0 182 669** |
| **DE-A- 3 618 517** | **GB-A- 1 087 415** |
| **US-A- 2 170 111** | **US-A- 2 189 397** |
| **US-A- 2 828 316** | **US-A- 4 958 032** |

- **CHEMICAL ABSTRACTS, vol. 103, no. 22, 2. Dezember 1985, Columbus, Ohio, US; abstract no. 179981r, TOHO CHEMICAL INDUSTRY CO. LTD. 'Amphoteric surface- active betaine compounds' Seite 100 ;Spalte 2 ;**
- **Kirk-Othmer, Encyclopedia of Chemical Technology, 3rd ed., Vol.22, page 833, 1979**
- **Kirk-Othmer, Encyclopedia of Chemical Technology, 3rd ed., Vol. 7, page 332, 1983**
- **Kirk-Othmer, Encyclopedia of Chemical Technology, 4th ed., Vol. 8, page 300, 1993**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.1/3.4

## Beschreibung

Die vorliegende Erfindung betrifft die Verwendung von Alkoxylierungsprodukten der allgemeinen Formel I

$$(R^1)_x — A [(B\text{-}O)_n — Z]_y \qquad I$$

in der die Variablen folgende Bedeutung haben:

A Sauerstoff oder eine -CO-O-Gruppe, wenn x und y für 1 stehen, oder Stickstoff, wenn x+y = 3 ist,

B Ethyliden oder 1,2-Propyliden;

Z eine der Gruppen

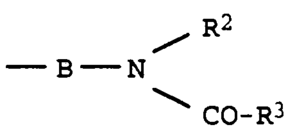 , 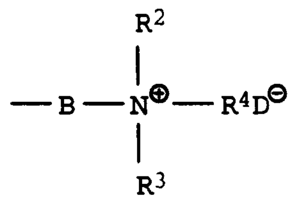 ,

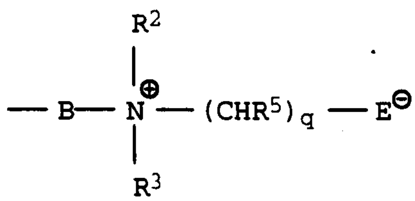 , 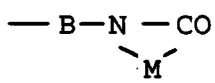 ,

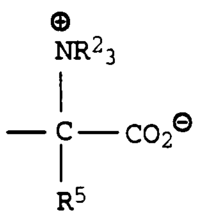 ;

$D^{\ominus}$ Formiat, Acetat, Propionat, Hydroxid;

$E^{\ominus}$ Carboxylat, Sulfonat;

M eine Brückengruppierung zur Vervollständigung eines Pyrrolidon-, Succinimid- oder Maleinimidrings als Gruppierung

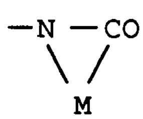

n 1 bis 50;

q 1 bis 4;

x, y 1 oder 2 mit der Maßgabe, daß x+y ≤ 3 ist;

$R^1$ $C_8$-$C_{30}$-Alkyl; daneben, wenn A für Sauerstoff steht: $C_4$-$C_{12}$-alkylsubstituiertes Phenyl;

$R^2$, $R^3$, $R^4$ Wasserstoff, Methyl oder Ethyl;

$R^5$ Wasserstoff oder Methyl;

zur Dispergierung feinteiliger Feststoffe in einem fließfähigen Medium.

Außerdem betrifft die Erfindung neue Alkoxilierungsprodukte der allgemeinen Formel II:

$$(R^1)_x - A - [(B-O)_n - Z]_y \qquad \text{II}$$

in der die Variablen folgende Bedeutung haben:

A Sauerstoff oder eine -CO-O-Gruppe, wenn x und y für 1 stehen, oder Stickstoff, wenn x+y = 3 ist,
B Ethyliden oder 1,2-Propyliden
Z eine der Gruppen

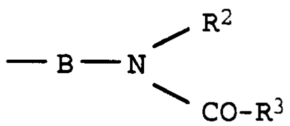 , 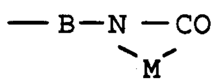 ,

M eine Brückengruppierung zur Vervollständigung eines Succinimid- oder Maleinimidrings als Gruppierung

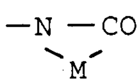 ,

n 1 bis 50;
x, y 1 oder 2 mit der Maßgabe, daß x+y ≤ 3 ist
$R^1$ $C_8$ - $C_{30}$-Alkyl; daneben, wenn A für Sauerstoff steht: $C_4$-$C_{12}$-alkylsubstituiertes Phenyl;
$R^2$, $R^3$ Wasserstoff, Methyl oder Ethyl.

Weiterhin betrifft die Erfindung ein Verfahren zur Herstellung der Alkoxilierungsprodukte der allgemeinen Formel II sowie Zubereitungen, welche die Verbindungen II enthalten.

Für eine Vielzahl technischer Verfahren ist es unerläßlich, feinteilige Pulver in stabile Dispersionen zu überführen. Zur Dispergierung in einem Medium, in dem die Pulver nicht löslich sind, werden Dispergatoren verwendet. Da sich die Anforderungen an Dispergatoren durch die große Zahl verschiedener Pulver und möglicher Dispergiermedien stark unterscheiden, sind unwirtschaftliche Einzellösungen für Dispergierprobleme, d.h. ein bestimmter Dispergator für eine spezielle Kombination von Pulver und Dispergiermedium, weit verbreitet.

Aus der GB-A 1 342 746 sind Polyester aus Hydroxycarbonsäuren und deren Verwendung als Dispergatoren für eine Reihe von Pigmenten in organischen Lösungsmitteln bekannt. Für eine Anwendung in Wasser sind diese Dispergatoren aber nicht geeignet.

GB-A 1 087 415 und JP-A 60 89 458 (Chem. Abstr. 103:179981r) lehren amphotere oberflächenaktive Alkoxilierungsprodukte mit Betainstruktur sowie deren Verwendung als Reinigungsmittel, in Shampoos oder in Geschirrspülmitteln.

Aus EP-A 182 669 sind oberflächenaktive Verbindungen vom Typ der quartären Ammoniumsalze bekannt, die für eine Vielzahl von Anwendungen, von Weichmachern für Gewebe über Antistatika bis hin zu Bioziden, geeignet sind.

US-A 4 958 032 lehrt die Anwendung N-substituierter γ-Butyrolactame, deren N-Substituent eine über eine 1,3-Propylidengruppe an den Lactanstickstoff gebundene Kette aus Ethoxi- und/oder Propoxieinheiten, die am anderen Ende von einer Ethergruppe terminiert wird, enthält, als Emulgator, Benetzungsmittel, Weichmacher, Verfilzungsverhinderer, Konditionsmittel, Komplexier- und Löslichkeitsvermittler.

Weiterhin sind aus der DE-A 40 26 965 gießfähige Formmassen bekannt, die neben sinterbaren Pulvern sowie organischen Lösungsmitteln aus der Reihe der Alkane, Ether, Ester und Ketone Dispergatoren der allgemeinen Formel I' enthalten.

$$(R^1)_x - A\,[(B\text{-}O)_n - Z']_y \qquad I'$$

Diese Verbindungen unterscheiden sich in der polaren Endgruppe Z' von den erfindungsgemäßen Verbindungen I.

Z' steht für Wasserstoff, einen Sulfonsäurerest, einen Phosphorsäurerest, eine Carboxylatgruppe, eine Aminogruppe oder eine Amidgruppe mit einer freien Aminokomponente.

Diese Dispergatoren sind für die genannten organischen Dispergiermedien gut geeignet, lassen aber in alkohol- und wasserhaltigen Medien zu wünschen übrig.

Neben einer homogenen Verteilung der Pulver in den Dispersionen soll ein Dispergator weiterhin einen möglichst hohen Füllgrad der Dispersionen mit dem jeweiligen Pulver erlauben, da die Verarbeitung (z.B. durch Mahlen) hochgefüllter Dispersionen in der Regel wirtschaftlicher ist als die niedriger gefüllter, solange die Viskosität bestimmte Grenzen nicht übersteigt.

Es bestand daher die Aufgabe, Verbindungen zu finden, die breite Dispergiereigenschaften bieten, d.h. viele verschiedene Pulver jeweils in verschiedenartigen Medien dispergieren. Weiterhin sollen sie einen hohen Füllgrad ermöglichen bzw. bei konstantem Füllgrad die Herstellung möglichst niedrigviskoser Zubereitungen erlauben.

Demgemäß wurden die eingangs definierten Alkoxylierungsprodukte der Formel II gefunden.

Weiterhin wurde ein Verfahren zur Herstellung der Verbindungen II gefunden. Außerdem wurden Zubereitungen gefunden, die neben feinteiligen Pulvern und fließfähigen Medien eine Verbindung II enthalten.

Ferner wurde die Verwendung der Verbindungen der Formel I als Dispergatoren für feinteilige Pulver in fließfähigen Medien gefunden.

Im einzelnen haben die Variablen und Gruppierungen in den Formeln I und II folgende Bedeutung:

A vorzugsweise Sauerstoff, daneben eine -CO-O-Gruppe, wenn x und y für 1 stehen; weiterhin Stickstoff, wenn $x+y = 3$ ist;

B vorzugsweise Ethyliden, daneben 1,2-Propyliden;

Z eine der Gruppen

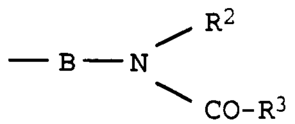 , 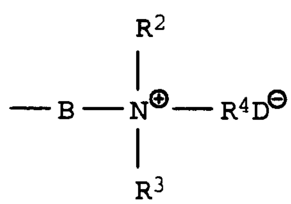 ,

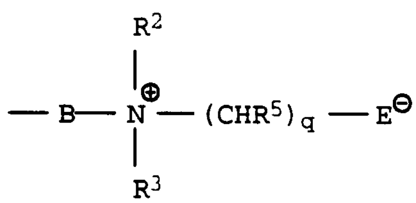 , 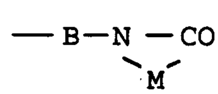 ,

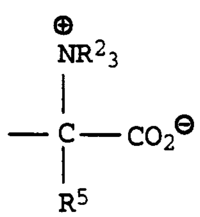 ;

wobei folgende Gruppen bevorzugt sind:

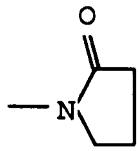 , 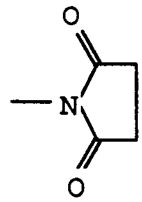 , 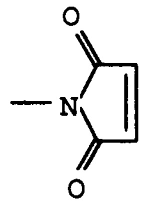 ,

$-CH_2CH_2N^{\oplus}R^2R^3-CH_2-CO_2^{\ominus}$; mit der Maßgabe, daß Z in Formel II ausschließlich für eine der Gruppen

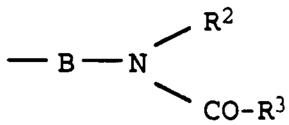 oder 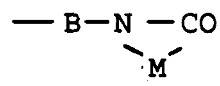

steht;

$D^{\ominus}$ vorzugsweise Acetat, aber auch Formiat, Propionat und Hydroxyd;

$E^{\ominus}$ vorzugsweise Carboxylat, daneben Sulfonat;

M eine Brückengruppierung zur Vervollständigung eines Pyrrolidon-, Succinimid- oder Maleinimidrings als Gruppierung

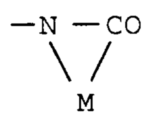

mit der Maßgabe, daß M in Formel II ausschließlich eine Brückengruppierung zur Vervollständigung eines Succinimid- oder Maleinimidrings darstellt;

| | |
|---|---|
| n | 1 bis 50, vorzugsweise 2 bis 25; |
| q | 1 bis 4, wovon 1 bevorzugt wird; |
| x, y | 1 oder 2 mit der Maßgabe, daß $x+y \leq 3$ ist; |
| $R^1$ | vorzugsweise eine $C_8$-$C_{30}$-, besonders eine $C_{12}$-$C_{18}$-Alkylgruppe, darunter besonders Gemische aus $C_{13}/C_{15}$-Oxoalkoholresten sowie $C_{12}/C_{14}$- und $C_{16}/C_{18}$-Fettalkoholresten; daneben im Falle, daß A für Sauerstoff steht, ein $C_4$-$C_{12}$-alkylsubstituierter Phenylrest, von denen tert.-Butyl-, iso-Octyl- und iso-Nonylphenyl bevorzugt werden; |
| $R^2$, $R^3$, $R^4$ | vorzugsweise Wasserstoff und Methyl, aber auch Ethyl; |
| $R^5$ | Wasserstoff, Methyl. |

Bei den Verbindungen I und II handelt es sich um Alkylenoxid- oder Polyalkylenoxidderivate mit den Resten $(R^1)_x$-A- und -Z als terminierende Gruppen.

Zweckmäßigerweise geht man zur Synthese von I oder II von aminierten Alkoxyalkoholen III

$$(R^1)_x - A \left[ (B\text{-}O)_n - B - NR^2R^3 \right]_y \qquad III$$

oder oxidierten Alkoxyalkoholen IV

$$(R^1)_x - A\ [(B\text{-}O)_n - CHR^5\text{-}COOH]_y \qquad IV$$

aus.

Bedeutet A Sauerstoff, erhält man die Verbindungen III durch Alkoxylierung eines Alkohols $R^1$-OH mit einem Alkylenoxid und weitere Aminierung, z.B. nach der Lehre der US-A-3 440 029. Eine vollständige Methylierung am Stickstoffatom ist gewünschtenfalls durch eine Umsetzung mit Ameisensäure und Formaldehyd möglich.

Bedeutet A die -CO-O-Gruppe, wird die Synthese von III analog zur oben beschriebenen Reaktion mit einer Carbonsäure $R^1$-COOH anstelle eines Alkohols durchgeführt.

Steht A für Stickstoff, werden aminierte Fettalkohole, die z.T. im Handel oder nach bekannten Methoden erhältlich sind, alkoxyliert und wie oben beschrieben aminiert. Dabei kann das Stickstoffatom zwei Alkylreste und somit eine Alkylenoxideinheit oder aber einen Alkylrest und zwei Alkylenoxideinheiten tragen.

Die Verbindungen IV sind durch Oxidation anstelle einer Aminierung der vorstehend beschriebenen Alkoxyalkohole erhältlich. Teilweise sind sie auch im Handel erhältlich.

In die so gewonnenen Zwischenverbindungen III bzw. IV werden durch Folgereaktionen die polaren Endgruppen Z eingeführt.

Im einzelnen wird dazu eine Verbindung III, in der mindestens einer der Reste $R^2$ und $R^3$ für Wasserstoff steht, in bekannter Weise mit einer Carbonsäure $R^3$-COOH zum Amid umgesetzt.

Die Umsetzung einer Verbindung III mit einer $C_1$-$C_3$-Carbonsäure unter weniger drastischen Reaktionsbedingungen als im vorstehenden Fall führt zu $C_1$-$C_3$-Carbonsäuresalzen.

Weiterhin können die Verbindungen III mit Chlorwasserstoffsäure zu den entsprechenden Chloriden umgesetzt werden, welche durch Reaktion mit Silberhydroxid zu Ammoniumhydroxiden umgesetzt werden können.

Erfindungsgemäße Betaine können erhalten werden, wenn eine Verbindung III mit einer $\omega$-Chlor-($C_2$-$C_4$)-carbonsäure oder deren Alkalimetallsalz reagiert.

Eine Verbindung III mit endständiger $NH_2$-Gruppe kann durch Umsetzung mit $\gamma$-Butyrolacton, Bernsteinsäureanhydrid bzw. Maleinsäureanhydrid in eine Verbindung I oder II überführt werden, in der

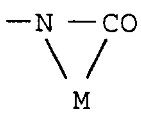

für eine N-Pyrrolidon-, N-Succinimid- bzw. N-Maleinimidgruppe steht.

Schließlich führt die $\alpha$-Halogenierung einer Verbindung IV und anschließende Substitution mit Ammoniak oder einem Amin gefolgt von einer Alkylierung am Stickstoff, z.B. mit einem Alkyliodid, zu einem Betain, in dem Z für -$CR^5(N^{\oplus}R^2_3)CO_2^{\ominus}$ steht.

Die Verbindungen I finden Verwendung zur Dispergierung feinteiliger Feststoffe in einem fließfähigen Medium.

Derartige Feststoffe sind neben anorganischen und organischen Pigmenten bevorzugt oxidische oder nichtoxidische keramische Pulver und Metallpulver.

Als oxidische Pulver sind u.a. MgO, $TiO_2$, $ZrO_2$, $CrO_2$, $Y_2O_3$, $Al_2O_3$ und $Fe_2O_3$ zu nennen, aber auch Mischoxide wie $BaTiO_3$, Aluminiumsilikate, stabilisierte Zirkonoxide und Ferrite.

Als nichtoxidische keramische Pulver kommen u.a. SiC, $Si_3N_4$, BN, AlN und WC in Betracht. Auch Graphit kommt in Betracht.

Beispiele für Metalle sind Fe, Ni, Cr, Mo und das Halbmetall Si.

Feinteilig bedeutet, daß die mittleren Korngrößen im allgemeinen 0,1 bis 50 $\mu$m betragen.

Fließfähige Medien umfassen Flüssigkeiten, die bei Raumtemperatur flüssig sind, wie Wasser oder organische Lösungsmittel wie Alkohole, Ether, Etheralkohole, Ester, aliphatische und aromatische Kohlenwasserstoffe, halogenierte Kohlenwasserstoffe, Amine, Amide und Nitroverbindungen. Selbstverständlich können auch Mischungen dieser Stoffe verwendet werden.

Weiterhin kommen Stoffe in Betracht, die erst bei höherer Temperatur als Raumtemperatur fließfähig werden, wie

Wachse und thermoplastische Polymere, z.B. Polymethylmethacrylat, Polystyrol, Polyamide, Polyoxymethylen, Polyethylen und Polyester. Es kommen auch Lösungen von Monomeren oder Polymeren in Betracht.

Aus den oben genannten feinteiligen Feststoffen, den fließfähigen Medien sowie Verbindungen der Formel II sind erfindungsgemäße Zubereitungen erhältlich.

Die Zubereitungen oder Dispersionen feinteiliger Feststoffe in einem fließfähigen Medium enthalten in der Regel:

| | |
|---|---|
| 20 - 99,8 | Gew.-% feinteilige Feststoffe, |
| 0,1 - 80 Gew.-% | fließfähiges Medium und |
| 0,1 - 20 Gew.-% | Verbindung I oder II, vorzugsweise 0,1-5 Gew.-%. |

Weiterhin können die Zubereitungen oder Dispersionen für die jeweilige Verwendung übliche Hilfs- und Zusatzstoffe enthalten, z.B. Weichmacher, Wasserrückhaltestoffe, Entformungshilfen, UV-Stabilisatoren und Korrosionsinhibitoren.

Die Herstellung der Zubereitungen oder Dispersionen erfolgt in an sich bekannter Weise. So hat es sich besonders bewährt, im kleinen Maßstab die Dispergierung mit Ultraschallwellen vorzunehmen, indem Dispergiermedium und Dispergator in einem Ultraschallbad mit dem Feststoff versetzt werden.

Für größere Ansätze wird man zweckmäßigerweise auf die Vermahlung, z.B. in einer Kugelmühle, zurückgreifen oder Kneter verwenden. Hochgefüllte Kunststoffe können vorteilhaft in einem Extruder hergestellt werden. Dabei können Pulver und Dispergator getrennt oder Granulate aus Pulver, das mit dem Dispergator beschichtet ist, verwendet werden. Die Granulate können durch Vermischen eines Pulvers und Dispergators in einem Lösungsmittel und anschließendes Entfernen des Lösungsmittels bis zur Trockne hergestellt werden.

Die Verbindungen I und II erlauben die Dispergierung verschiedener Pulver in polaren, mittelpolaren und schwach polaren Lösungsmitteln. Dabei werden hohe Füllgrade erzielt und die Dispersionen weisen eine relativ niedrige Viskosität bei konstanten Füllgraden auf.

Beispiele

1. Herstellung der Verbindungen I und II

Beispiel 1 (Dispergator A) $(C_{13/15}\text{-Alkyl})\text{-O-}(CH_2CH_2O)_6\text{-}CH_2CH_2N^{\oplus}H_2(CH_3)CH_3CO_2^{\ominus}$

Ein ethoxylierter $C_{13}$-$C_{15}$-Oxoalkohol (Verhältnis von $C_{13}$ zu $C_{15}$ 2:1) mit einem Ethoxylierungsgrad von 7 wurde mit Methylamin aminiert. Zu 100 g des so erhaltenen Aminierungsproduktes wurden 11,2 g Essigsäure gegeben. Überschüssige Essigsäure wurde bei 80°C im Vakuum entfernt und es verblieb eine gelbe Flüssigkeit.

Beispiel 2 (Dispergator B) $(C_{13/15}\text{-Alkyl})\text{-O-}(CH_2CH_2O)_6\text{-}CH_2CH_2N^{\oplus}(CH_3)_2CH_2CO_2^{\ominus}$

100 g des unter Beispiel 1 hergestellten Aminierungsproduktes wurden mit 43 g konz. Ameisensäure und 16,8 g einer 40 gew-%igen Formalinlösung 2 h unter Rückflußkühlung erhitzt, neutralisiert, azeotrop mit Toluol destilliert und filtriert.

Das Lösungsmittel wurde entfernt.

50 g der so erhaltenen schwach gelben Flüssigkeit wurde 5 h mit 13,8 g einer wäßrigen 50 gew.-%igen Lösung des Natriumsalzes der Chloressigsäure unter Rückflußkühlung erhitzt. Nach Abtrennung des Wassers und Salzes verblieb eine braune viskose Flüssigkeit.

Beispiel 3 (Dispergator C) $(C_{16/18}\text{-Alkyl})\text{-O-}(CH_2CH_2O)_{10}\text{-}CH_2CH_2N^{\oplus}(CH_3)_2CH_2CO_2^{\ominus}$

In Analogie zu Beispiel 1 wurde ein ethoxylierter $C_{16}$-$C_{18}$-Fettalkohol (Verhältnis von $C_{16}$ zu $C_{18}$ 0,5:1) mit einem Ethoxylierungsgrad von 11 mit Methylamin aminiert und analog zu Beispiel 2 methyliert und zum Betain umgesetzt.

Beispiel 4 (Dispergator D)

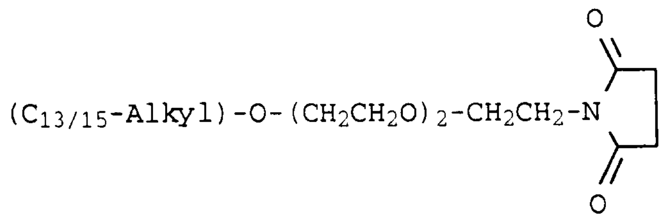

Ein $C_{13}$-$C_{15}$-Oxoalkohol (Verhältnis von $C_{13}$ zu $C_{15}$ 2:1) mit einem durchschnittlichen Ethoxylierungsgrad von 3 wurde mit einem Überschuß Ammoniak aminiert. Zu 100 g des so erhaltenen Aminierungsproduktes wurden 29 g Bernsteinsäureanhydrid gegeben. Bei 150°C wurde 2 h Wasserstrahlvakuum angelegt. Es wurde eine dunkelbraune Flüssigkeit erhalten.

Beispiel 5 (Dispergator E)

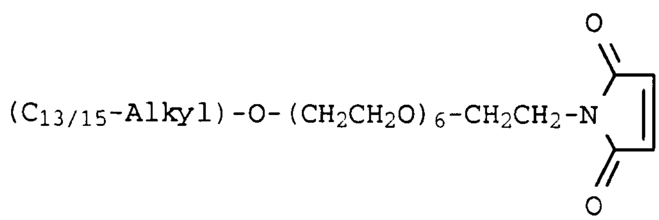

Ein ethoxylierter $C_{13}$-$C_{15}$-Fettalkohol (Verhältnis von $C_{13}$ zu $C_{15}$ 2:1) mit einem Ethoxyllerungsgrad von 7 wurde mit Ammoniak aminiert.

100 g der so erhaltenen farblosen Flüssigkeit wurden mit 19 g Maleinsäureanhydrid 2 h auf 160°C erhitzt, wobei Wasserstrahlvakuum angelegt wurde. Es wurde eine dunkelbraune Flüssigkeit erhalten.

Beispiel 6 (Dispergator F)

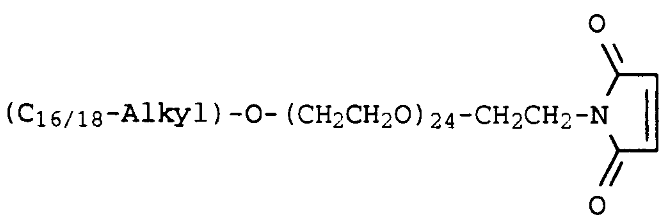

Ein ethoxylierter $C_{16}$-$C_{18}$-Fettalkohol (Verhältnis $C_{16}$ zu $C_{18}$ 0,5:1) mit einem Ethoxylierungsgrad von 25 wurden mit Ammoniak aminiert.

100 g des so erhaltenen farblosen Wachses wurden mit 7,2 g Maleinsäureanhydrid in Analogie zu Beispiel 5 umgesetzt. Es wurde ein goldbraunes Wachs erhalten.

Beispiel 7 (Dispergator G)

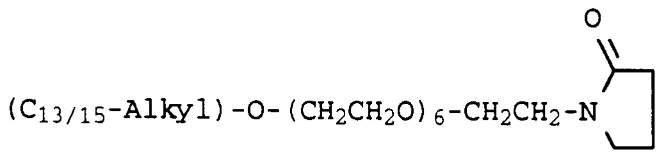

100 g des Aminierungsproduktes nach Beispiel 5 wurden mit 17 g γ-Butyrolacton 5 h auf 180°C erhitzt. Es wurde eine hellbraune Flüssigkeit erhalten.

Beispiel 8 (Dispergator H)

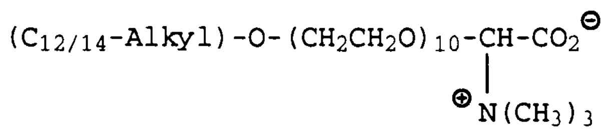

100 g eines ethoxylierten $C_{12}$-$C_{14}$-Fettalkohols (Verhältnis $C_{12}$ zu $C_{14}$ 2:1) mit einem Ethoxylierungsgrad von 10 mit einer endständigen Carboxylgruppe wurde mit 1 g rotem Phosphor und 100 g Brom versetzt. Nach 1 Woche bei 50°C wurden alle flüchtigen Bestandteile entfernt.
Der Rückstand wurde mit 100 ml einer 20 gew.-%igen wäßrigen Ammoniaklösung versetzt. Nach 4 Tagen wurde das Wasser azeotrop entfernt, filtriert und flüchtige Bestandteile wurden abgezogen. Das Produkt wurde in 200 ml Nitromethan mit 100 g Methyliodid versetzt. Nach 30 min wurden bei 80°C alle flüchtigen Bestandteile entfernt. Es verblieb eine hellbraune Paste.

2. Hochgefüllte Dispersionen

50 ml Lösungsmittel und die unten angegebene Menge Dispergator wurden unter Rühren vorgelegt und portionsweise unter Beschallung im Ultraschallbad mit den unten angegebenen Pulvern versetzt. Die Zugabe wurde abgebrochen, wenn der Ansatz nicht mehr rührbar war.

Als Vergleich wurde ein Dispergator folgender Struktur verwendet:

$$(C_{13/15}\text{-Alkyl})\text{-O-}(CH_2CH_2O)_7\text{-H}$$

wobei das Verhältnis $C_{13}$ zu $C_{15}$ 2:1 betrug. Vom Vergleichsdispergator wurden die gleichen Mengen wie vom erfindungsgemäßen Dispergator eingesetzt.

Je größer die in die Dispersion einarbeitbare Menge Pulver ist, desto wirksamer ist der verwendete Dispergator.

Folgende Ansätze wurden gemacht:

| Pulver | Korngröße [μm] | Dispergatormenge [g] |
|---|---|---|
| $ZrO_2$ (5 Gew.-% $Y_2O_3$) | 0,3 | 6 |
| SiC | 0,6 | 3,2 |
| $Y_2O_3$ | 0,4 | 5 |
| $Si_3N_4$ | 0,7 | 3 |
| $Al_2O_3$ | 0,7 | 4 |

2.1 Schwach polare Lösungsmittel

In der Tabelle sind die Pulvermengen angegeben, die in 50 ml Lösungsmittel der angegebenen Dispergatormenge rührbar waren.

| Lösungsmittel | Pulver | Dispergator | Pulvermenge [g] | |
|---|---|---|---|---|
| | | | erfindungsgemäß | Vergleich |
| n-Octan | SiC | A | 160 | 155 |
| n-Octan | $Y_2O_3$ | C | 190 | 150 |
| Toluol | SiC | E | 160 | 70 |
| Toluol | $Y_2O_3$ | E | 205 | 105 |

2.2 Mittelstark polare Lösungsmittel

| Lösungsmittel | Pulver | Dispergator | Pulvermenge [g] | |
|---|---|---|---|---|
| | | | erfindungsgemäß | Vergleich |
| Dimethylacetamid | $Y_2O_3$ | E | 195 | 110 |
| THF | $Y_2O_3$ | C | 180 | 115 |
| Butylacetat | $ZrO_2$ | E | 250 | 115 |
| | $Y_2O_3$ | E | 195 | 65 |

2.3 Polare Lösungsmittel

| Lösungsmittel | Pulver | Dispergator | Pulvermenge [g] | |
|---|---|---|---|---|
| | | | erfindungsgemäß | Vergleich |
| Wasser | $Si_3N_4$ | C | 145 | 110 |
| | $Al_2O_3$ | C | 145 | 115 |
| n-Butanol | SiC | E | 155 | 90 |
| | SiC | H | 160 | 90 |
| | $Y_2O_3$ | E | 195 | 75 |
| iso-Propanol | $Al_2O_3$ | E | 200 | 160 |
| | SiC | B | 150 | 120 |
| | $Y_2O_3$ | E | 165 | 130 |

2.4 Viskositätsmessung

In diesen Versuchen wurden 200 g $Al_2O_3$-Pulver der mittleren Korngröße 0,7 µm zu 50 ml Lösungsmittel und 4 g erfindungsgemäßem Dispergator gegeben und durch Rühren im Ultraschallbad dispergiert. Bei einer Schergeschwindigkeit von 1000 $s^{-1}$ wurde mit einem Rotationsviskosimeter die Viskosität bestimmt.

Je niedriger bei gleichem Füllgrad der Dispersion die Viskosität ist, desto wirksamer ist der verwendete Dispergator.

| Lösungsmittel | Dispergator | Viskosität [mPa • s] | |
|---|---|---|---|
| | | erfindungsgemäß | Vergleich |
| n-Octan | G | 33 | 36 |
| Toluol | E | 34 | (122 g)* |
| THF | E | 78 | (115 g)* |
| | F | 32 | (115 g)* |
| Butylacetat | F | 43 | (85 g)* |
| | H | 39 | (85 g)* |

* nicht rührbar

2.5 Viskosität bei Extrusion

Es wurde ein Granulat I aus $ZrO_2$-Teilchen, die mit Dispergator E beschichtet waren, hergestellt. Dazu wurden 363 g $ZrO_2$-Pulver (stabilisiert mit 5,2 Gew.-% $Y_2O_3$; Korngröße 0,3 μm), 5,45 g Dispergator E und 140 ml n-Octan 2 h bei 2200 $min^{-1}$ in einer Rührwerkskugelmühle vermahlen. Die so erhaltene Dispersion wurde bei 100°C im Vakuum zur Trockne eingeengt.

300 g des Granulates wurde zu 67 g Polyoxymethylen, das bei 162°C in 300 ml Diethylenglykoldimethylether gelöst wurde, gegeben und in ca. 30 s im Vakuum unter Aufschäumen vom Lösungsmittel befreit. Das aufgeschäumte Produkt wurde zerkleinert.

Zur Viskositätsmessung wurde die Zubereitung in einen 170°C heißen Zylinder gegeben und bei konstanter Kolbengeschwindigkeit durch eine auswechselbare Düse extrudiert.

Die Schmelze aus dem Granulat I war durch eine Lochdüse mit 10 Löchern von 70 μm Durchmesser noch extrudierbar. Die so erzeugten Fasern erreichten eine Länge von mehreren Metern.

Ein Vergleichsgranulat II (mit dem Vergleichsdispergator in Analogie zu Granulat I hergestellt) war lediglich durch ein Loch von 2 mm Durchmesser extrudierbar.

**Patentansprüche**

**1.** Verwendung von Alkoxilierungsprodukten der allgemeinen Formel I:

$$(R^1)_x - A - [(B\text{-}O)_n - Z]_y \qquad I$$

in der die Variablen folgende Bedeutung haben:

A Sauerstoff oder eine -CO-O-Gruppe, wenn x und y für 1 stehen, oder Stickstoff, wenn x+y = 3 ist,
B Ethyliden oder 1,2-Propyliden
Z eine der Gruppen

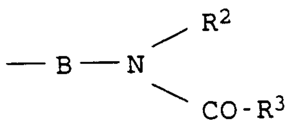
,
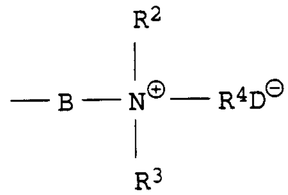
,

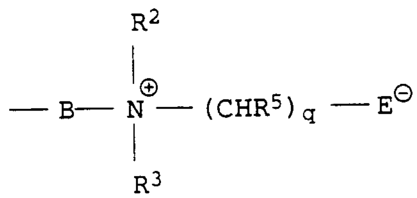
,
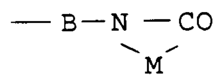
,

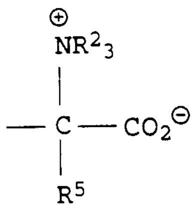
;

| | |
|---|---|
| $D^-$ | Formiat, Acetat, Propionat, Hydroxid; |
| $E^-$ | Carboxilat, Sulfonat; |
| M | eine Brückengruppierung zur Vervollständigung eines Pyrrolidon-, Succinimid- oder Maleinimidrings als Gruppierung |

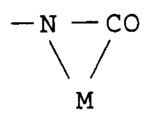

| | |
|---|---|
| n | 1 bis 50; |
| q | 1 bis 4; |
| x, y | 1 oder 2 mit der Maßgabe, daß $x+y \leq 3$ ist |
| $R^1$ | $C_8$ - $C_{30}$-Alkyl; daneben, wenn A für Sauerstoff steht: $C_4$-$C_{12}$-alkylsubstituiertes Phenyl; |
| $R^2$, $R^3$, $R^4$ | Wasserstoff, Methyl oder Ethyl; |
| $R^5$ | Wasserstoff oder Methyl, |

zur Dispergierung feinteiliger Feststoffe in einem fließfähigen Medium.

2. Verwendung von Alkoxilierungsprodukten der allgemeinen Formel I, in denen B für den 1,2-Ethylidenrest steht, zur Dispergierung feinteiliger Feststoffe in einem fließfähigen Medium.

3. Verwendung von Alkoxilierungsprodukten der allgemeinen Formel I, in denen die Gruppe

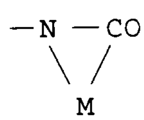

für den Pyrrolidon-, den N-Succinimid- oder den N-Maleinimidring steht, zur Dispergierung feinteiliger Feststoffe in einem fließfähigen Medium.

**4.** Verwendung von Alkoxilierungsprodukten der allgemeinen Formel I, in denen die Gruppe Z für den Rest

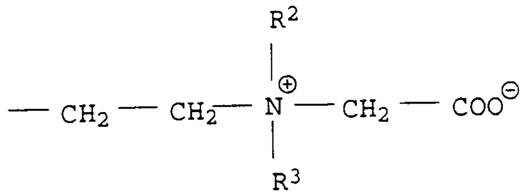

steht, zur Dispergierung feinteiliger Feststoffe in einem fließfähigen Medium.

**5.** Als Dispergatoren geeignete Alkoxilierungsprodukte der allgemeinen Formel II:

$$(R^1)_x - A - [(B\text{-}O)_n - Z]_y \qquad \text{II}$$

in der die Variablen folgende Bedeutung haben:

| | |
|---|---|
| A | Sauerstoff oder eine -CO-O-Gruppe, wenn x und y für 1 stehen, oder Stickstoff, wenn $x+y = 3$ ist, |
| B | Ethyliden oder 1,2-Propyliden |
| Z | eine der Gruppen |

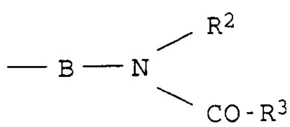 , 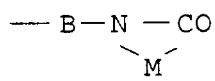 ,

| | |
|---|---|
| M | eine Brückengruppierung zur Vervollständigung eines Succinimid- oder Maleinimidrings als Gruppierung |

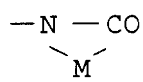 ,

| | |
|---|---|
| n | 1 bis 50; |
| x, y | 1 oder 2 mit der Maßgabe, daß $x+y \leq 3$ ist |
| $R^1$ | $C_8$ - $C_{30}$-Alkyl; daneben, wenn A für Sauerstoff steht: $C_4$-$C_{12}$-alkylsubstituiertes Phenyl; |
| $R^2$, $R^3$ | Wasserstoff, Methyl oder Ethyl. |

**6.** Alkoxilierungsprodukte nach Anspruch 5, in denen B für den 1,2-Ethylidenrest steht.

**7.** Alkoxilierungsprodukte nach den Ansprüchen 5 oder 6, in denen die Gruppe

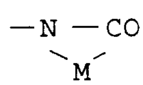

für den N-Succinimid- oder den N-Maleinimidring steht.

**8.** Zubereitungen, die neben feinteiligen Feststoffen und fließfähigen Medien Alkoxilierungsprodukte der allgemeinen Formel II gemäß einem der Ansprüche 5, 6 oder 7 enthalten.

**9.** Zubereitungen nach Anspruch 8, in denen der Feststoff ein Metallpulver, ein oxidisches oder ein nichtoxidisches keramisches Pulver ist.

**10.** Zubereitungen nach Ansprüchen 8 oder 9, in denen das fließfähige Medium ein Lösungsmittel und/oder ein thermoplastischer Kunststoff ist.

**11.** Verfahren zur Herstellung der Alkoxilierungsprodukte der allgemeinen Formel II, dadurch gekennzeichnet, daß man aminierte Alkoxialkohole der allgemeinen Formel III

$$(R^1)_x - A - [(B\text{-}O)_n - NR^2R^3]_y \qquad \text{III}$$

in der die Substituentensymbole die in Formel I und II definierten Bedeutungen haben, in an sich bekannter Weise

- für den Fall, daß mindestens einer der Reste $R^2$ und $R^3$ für Wasserstoff steht, mit einer Carbonsäure oder ihren funktionellen Derivaten zu einem Amid, umsetzt, oder
- für den Fall, daß R2 und R3 für Wasserstoff stehen, mit Bernsteinsäureanhydrid oder Maleinsäureanhydrid zu Verbindungen umsetzt, in denen

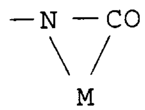

für eine N-Succinimid- oder N-Maleinimidgruppe steht.

## Claims

**1.** The use of alkoxylation products of the formula I

$$(R^1)_x - A - [(B\text{-}O)_n - Z]_y \qquad \text{I}$$

where

| | |
|---|---|
| A | is oxygen or a -CO-O- group when x and y are each 1 or is nitrogen when x+y is 3, |
| B | is ethylidene or 1,2-propylidene, |
| Z | is one of the groups |

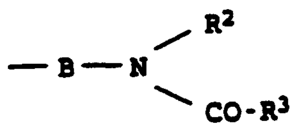 , 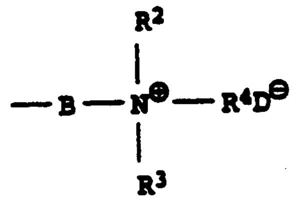 ,

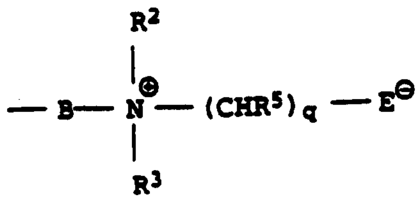 , 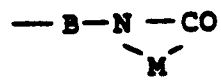 ,

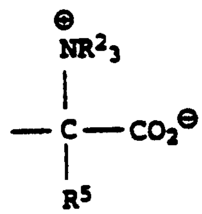 ,

| | |
|---|---|
| $D^-$ | is formate, acetate, propionate or hydroxide, |
| $E^-$ | is carboxylate or sulfonate, |
| M | is a bridge group for completing a pyrrolidone, succinimide or maleimide ring as group |

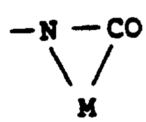

| | |
|---|---|
| n | is from 1 to 50, |
| q | is from 1 to 4, |
| x and y | are each 1 or 2, with the proviso that $x+y \leq 3$ , |
| $R^1$ | is $C_8$-$C_{30}$-alkyl or, when A is oxygen, $C_4$-$C_{12}$-alkyl-substituted phenyl, |
| $R^2$, $R^3$ and $R^4$ | are each hydrogen, methyl or ethyl and |
| $R^5$ | is hydrogen or methyl, for dispersing finely divided solids in a flowable medium. |

2. The use of alkoxylation products of the formula I where B is 1,2-ethylidene for dispersing finely divided solids in a flowable medium.

3. The use of alkoxylation products of the formula I where the group

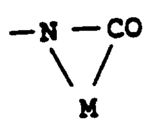

is the pyrrolidone, the N-succinimide or the N-maleimide ring for dispersing finely divided solids in a flowable medium.

4. The use of alkoxylation products of the formula I where Z is a radical

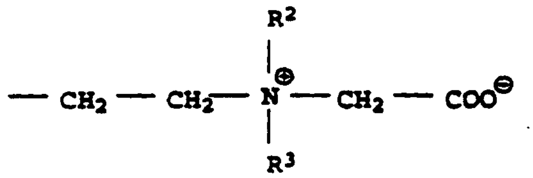

for dispersing finely divided solids in a flowable medium.

5. An alkoxylation product suitable as a dispersant and of the formula II

$$(R^1)_x - A - [(B\text{-}O)_n - Z]_y \qquad \text{II}$$

where

| | |
|---|---|
| A | is oxygen or a -CO-O- group when x and y are each 1 or is nitrogen when x+y is 3, |
| B | is ethylidene or 1,2-propylidene, |
| Z | is one of the groups |

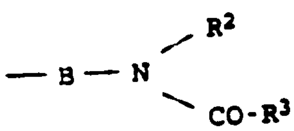 , 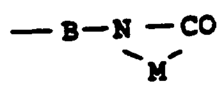 ,

| | |
|---|---|
| M | is a bridge group for completing a succinimide or maleimide ring as group |

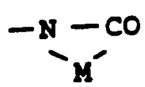 ,

| | |
|---|---|
| n | is from 1 to 50, |
| x and y | are each 1 or 2, with the proviso that $x+y \leq 3$ , |
| $R^1$ | is $C_8$-$C_{30}$-alkyl or, when A is oxygen, $C_4$-$C_{12}$-alkyl-substituted phenyl and |
| $R^2$ and $R^3$ | are each hydrogen, methyl or ethyl. |

6. An alkoxylation product as claimed in claim 5, in which B is 1,2-ethylidene.

7. An alkoxylation product as claimed in claim 5 or 6, in which the group

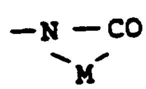

is the N-succinimide or the N-maleimide ring.

8. A formulation which, in addition to a finely divided solid and a flowable medium, contains an alkoxylation product of the formula II as claimed in any of claims 5, 6 or 7.

9. A formulation as claimed in claim 8, in which the solid is a metal powder or an oxidic or nonoxidic ceramic powder.

**10.** A formulation as claimed in claim 8 or 9, in which the flowable medium is a solvent and/or a thermoplastic.

**11.** A process for the preparation of an alkoxylation product of the formula II, wherein an aminated alkoxyalcohol of the formula III

$$(R^1)_x - A - [(B\text{-}O)_n - NR^2R^3]_y \qquad \text{III}$$

where the substituent symbols have the meanings defined for the formulae I and II, is reacted in a manner known per se

- where at least one of the radicals $R^2$ and $R^3$ is hydrogen, with a carboxylic acid or one of its functional derivatives to give an amide, or
- where $R^2$ and $R^3$ are each hydrogen, with succinic anhydride or maleic anhydride to give a compound in which

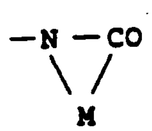

is a N-succinimide or a N-maleimide group.

**Revendications**

**1.** Utilisation de produits d'alcoxylation de formule générale I

$$(R^1)_x - A - [(B\text{-}O)_n - Z]_y \qquad \text{I}$$

dans laquelle les symboles ont les significations suivantes :

A : l'oxygène ou un groupe -CO-O lorsque x et y sont égaux à 1, ou l'azote lorsque x + y = 3 ,

B : un groupe éthylidène ou 1,2-propylidène

Z : l'un des groupes

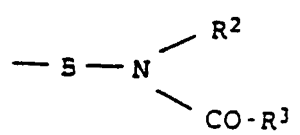
,
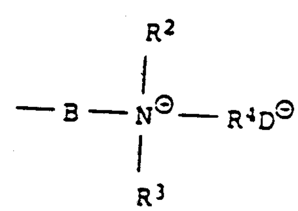

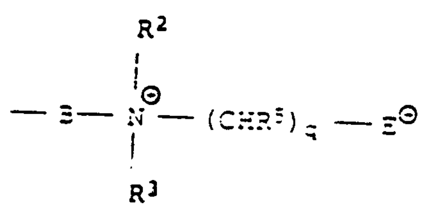
,
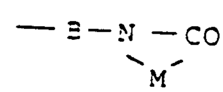

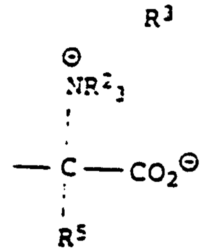
;

$D^-$ : un radical formiate, acétate, propionate, hydroxyde ;

$E^-$ : un radical carboxylate, sulfonate;

M : un pont permettant de compléter un cycle pyrrolidone, succinimide ou maléimide sous la forme d'un groupement

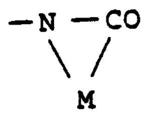

n : 1 à 50 ;

q : 1 à 4 ;

x, y : 1 ou 2, sous réserve que $x + y \leq 3$

$R^1$ : un groupe alkyle en C8-C30 ; ou encore, lorsque A représente l'oxygène : un groupe phényle à susbtituant alkyle en C4-C12 ;

$R^2$, $R^3$, $R^4$ : l'hydrogène, des groupes méthyle ou éthyle ;

$R^5$ : l'hydrogène ou un groupe méthyle, pour la dispersion de substances solides en fines particules dans un milieu fluide.

2. Utilisation de produits d'alcoxylation de formule générale I dans laquelle B représente le groupe 1,2-éthylidène pour la dispersion de matières solides en fines particules dans un milieu fluide.

3. Utilisation de produits d'alcoxylation de formule générale I dans laquelle le groupe

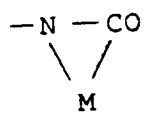

représente un cycle pyrrolidone, N-succinimide ou N-maléimide, pour la dispersion de substances solides en fines particules, dans un milieu fluide.

4. Utilisation de produit d'alcoxylation de formule générale I dans laquelle Z représente le groupe

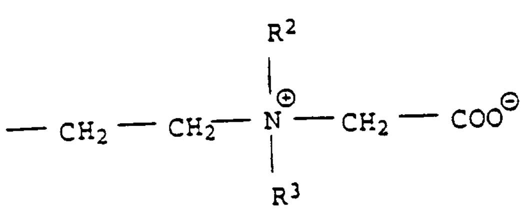

pour la dispersion de substances solides en fines particules dans un milieu fluide.

5. Produits d'alcoxylation, convenant à l'utilisation en tant qu'agents dispersant, qui répondent à la formule générale II

$$(R^1)_x - A - [(B-O)_n - Z]_y \qquad II$$

dans laquelle les symboles ont les significations suivantes :

A l'oxygène ou un groupe -CO-O, lorsque x et y sont égaux à 1 ou l'azote lorsque $x + y = 3$ ,

B un groupe éthylidène ou 1,2-propylidène,

Z l'un des groupes

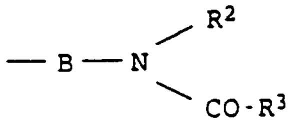
,
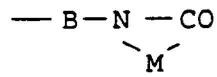

M un pont permetttant de compléter un cycle succinimide ou maléimide sous le forme d'un groupement

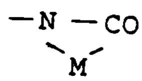

n 1 à 50 ;

x, y 1 ou 2 sous réserve que $x + y \leq 3$ ,

$R^1$ un groupe alkyle en C8-C30 ou encore, lorsque A représente l'oxygène : un groupe phényle à substituant alkyle en C4-C12 ;

$R^2$, $R^3$ l'hydrogène, des groupes méthyle ou éthyle

**6.** Produits d'alcoxylation selon revendication 5, pour lesquels B représente un groupe 1,2-éthylidène.

**7.** Produits d'alcoxylation selon les revendication 5 ou 6, pour lesquels le groupe

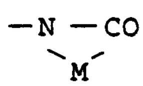

consiste en un cycle N-succinimide ou N-maléimide.

**8.** Composition contenant, avec des substances solides en fines particules et des milieux fluides, des produits d'alcoxylation de formule générale II selon une des revendications 5, 6 ou 7.

**9.** Compositions selon revendication 8, dans lesquelles la substance solide est une poudre métallique, une poudre céramique du type oxyde ou non.

**10.** Compositions selon les revendications 8 ou 9, dans lesquelles le milieu fluide est un solvant et/ou une résine synthétique thermoplastique.

**11.** Procédé de préparation des produits d'alcoxylation de formule générale II, caractérisé par le fait que, partant d'alcoxyalcools aminés de formule générale III

$$(R^1)_x - A - [(B\text{-}O)_n - NR^2R^3]_y \qquad III$$

dans laquelle les symboles ont les signification indiquées en référence aux formules I et II,

- dans le cas où l'un au moins des symboles $R^2$ et $R^3$ représente l'hydrogène, on fait réagir de manière connue en soi avec un acide carboxylique ou l'un de ses dérivés fonctionnels, avec formation d'un amide, ou bien
- dans le cas où $R^2$ et $R^3$ représentent l'hydrogène, on fait réagir de manière connue en soi avec l'anhydride succinique ou l'anhydride maléique, avec formation de composés lesquels

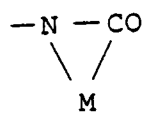

représente un groupe N-succinimide du N-maléimide.